# EUROPEAN PATENT SPECIFICATION

(11) **EP 0 500 763 B1**
(45) Date of publication and mention of the grant of the patent: **26.02.1997**
(21) Application number: 90917742.0
(22) Date of filing: 30.10.1990
(51) Int. Cl.: B01J 29/40

(54) **ZEOLITE L**
ZEOLITH
ZEOLITE L

(30) Priority: 30.10.1989 GB 8924410
(43) Date of publication of application: 02.09.1992
(73) Proprietor: EXXON RESEARCH AND ENGINEERING COMPANY, Florham Park, New Jersey 07932-0390 (US)
(72) Inventor: VERDUIJN, Johannes, Petrus, NL-3202 XL Spijkenisse (NL)
(74) Representative: Northover, Robert Frank
(86) International application number: US9006306
(87) International publication number: WO9106367

(56) References cited:
- EP-A- 0 167 755
- EP-A- 0 219 354
- EP-A- 0 280 513
- EP-A- 0 284 206
- US-A- 4 680 280

## Description

### FIELD OF THE INVENTION

The present invention relates to a zeolite of the L type, and a process for its production. This zeolite is a good catalyst base for a variety of organic reactions, especially hydrocarbon conversions, and may be regenerated after use.

### BACKGROUND OF THE INVENTION

Zeolite L has been known for some time as an adsorbent and in US-A-3216789 is described as an aluminosilicate of the formula:

0.9 -1.3M_{2/n}O : Al₂O₃ : 5.2 - 6.9 SiO₂ : yH₂O

(where M is an exchangeable cation of valency n and y is from 0 to 9) having a characteristic X-ray diffraction pattern.

EP-A-96479 describes a zeolite L which is particularly useful as a catalyst base in hydrocarbon conversions such as aromatization. The zeolite comprises crystallites in the form of cylinders with a mean diameter of at lest 0.1 micron, preferably at least 0.5 micron and with an aspect ratio (ratio of cylinder length to diameter) of at least 0.5. The gel from which the zeolite is obtained comprises the following ratios of components: 2.4 to 3.0 moles K₂O, 0.6 to 1.3 moles Al₂O₃, 8 to 12 moles of SiO₂ and 120 to 240 moles H₂O. A particularly preferred gel has the following compositions:
2.62K₂O : Al₂O₃ : 10SiO₂ : 16OH₂O.

EP-A-167755 discloses a similar zeolite L, but disc shaped having an aspect ratio of less than 0.5.

The potassium form of zeolite L, hereinafter identified as zeolite KL, may also contain caesium, as described in EP-A-323892.

Typically the zeolite is loaded with one or more metals such as platinum, tin, germanium, rhenium or iridium, particularly platinum, to prepare the desired catalyst.

New forms of zeolite KL are sought which are particularly useful as a catalyst base for aromatization and which permit regeneration of spent catalyst. Imperfections in the zeolite channels result in poor utilization of Pt, poor maintenance of catalyst activity, and undesirable secondary reactions. To improve the properties of such a zeolite the channel length of the zeolite should be decreased to well below one micron, but at the same time the surface area of the zeolite crystal should be maintained as large as is practicable, and the crystals should be well-formed i.e. without a significant level of crystal imperfections.

These features are present if well-formed zeolite crystals can be made in the form of very flat cylinders. The present invention provides a zeolite whose crystals have the requisite properties; the present invention also provides a process for producing such zeolites.

### SUMMARY OF THE INVENTION

Accordingly the present invention provides a process for the preparation of a zeolite of the KL-type in which the crystals are cylindrical and have an average length of 0.6 microns or less and an average length:diameter ratio of less than 0.5 and have substantially flat basal planes, and also provides such zeolites per se where the average length:diameter ratio is less than 0.5, and the average height:length ratio is 1 to 1.2.

It is believed that in such a zeolite the flatness of the basal planes is an indication of the intrinsic quality of the crystals and the shortness of the crystal length makes for less meandering channels.

### DESCRIPTION OF PREFERRED EMBODIMENTS

The cylindrical crystal particles are substantially in the form of cylinders of circular cross-section, and preferably substantially in the form of right circular cylinders where the base is normal to the cylinder axis.

The crystals are coin or hockeypuck shaped and have a relatively large diameter and short length. The "length" of a crystal is a measurement of the outer edge of the crystal perpendicular to the basal plane containing the diameter. The length is typically 0.1 to 0.6 preferably 0.1 to 0.3 microns and the diameter is generally 0.3 to 1.5 microns preferably 0.4-1.0 microns. When the length/diameter ratio is 0.2-0.5 the crystal shape is termed herein as "hockeypuck". When the ratio is less than 0.2 the shape is termed herein as "coin".

The crystals thus possess the advantages of a short channel length and of a relatively large diameter which gives increased selectivity and/or yield when it is used as a base for a catalyst. The average length of time over which the catalyst remains active i.e. the run length of the process before the catalyst requires regeneration is longer with the present form of zeolite than with previous zeolites L of larger channel length and crystal size. Another advantage is that crystals prepared by the present invention are easy to recover from the synthesis magma.

The crystals also have microscopically flat basal planes. This is an indication that the crystals are well-formed and have an acceptably low level of crystal imperfections. A measure of flatness is the ratio of height:length, where the height is the longest measurement in the same direction as the length. Thus if the basal plane contains raised steps or terraces the maximum measurement of height of the crystal will be greater than the measurement of the length. If the basal planes are flat the height:length ratio will be 1. The height:length ratio of the crystals should be as close as possible to 1, but a ratio of up to 1.2 may be tolerated.

The zeolites prepared by the invention are preferably aluminosilicates and will be described hereinafter in terms of aluminosilicates, though other elemental substitutions are possible, for example aluminium may be substituted by gallium, boron, iron and similar trivalent elements, and silicon may be substituted by elements such as germanium or phosphorus.

Preferably the zeolite synthesis mixture comprises water, a source of divalent cation, a source of K₂O, a source of SiO₂ and a source of alumina. The divalent cation may be a cation of nickel, magnesium, calcium, barium, cobalt, manganese, zinc, copper or tin. Magnesium and barium have each been found to be particularly effective when included in the synthesis mixture for the zeolite. Initial results show that cobalt-containing zeolites are comparable with magnesium or barium-containing zeolites.

The proportions of the materials in the synthesis mixture may be adjusted to obtain the necessary crystal morphology. Preferably the synthesis mixture should contain sources which provide a molar ratio of K₂O/SiO₂ of 0.20 - 0.35 more preferably 0.24 - 0.30.

Preferably the mixture should contain sources which provide a molar ratio of SiO₂/Al₂O₃ of 15-160, more preferably 20-40, and a molar ratio of H₂O/K₂O of 45-70, more preferably 50-65.

The ratios are, as is usual with zeolite synthesis mixtures, interdependent. For example, if a high SiO₂/Al₂O₃ ratio is used, then a high K₂O/SiO₂ ratio should also be used to obtain the necessary alkalinity.

Thus the process preferably prepares a crystallisation product from a mixture comprising q moles of water, a divalent cation, a source of m moles of K₂O, a source of n moles of SiO₂ and a source of p moles of Al₂O₃ where m:n is 0.2 to 0.35 and n:p is 15 to 160 and q:m is 45 to 70. More preferably m:n is 0.24-0.30, n:p is 20-40 and q:m is 50-65.

A typical ratio of the synthesis mixture is e.g. 2.65 K₂O/0.5 Al₂O₃/10 SiO₂/160 H₂O, and a suitable quantity of divalent cation.

Increasing the proportion of alumina tends to increase the ratio of length to diameter, and also to increase the tendency for the contaminant, zeolite W, to form. Increasing the proportion of H₂O also has this effect.

Increasing the proportion of SiO₂ congruently increases the dimensions of the crystals produced, and also increases the tendency for undesirable amorphous byproducts to form. Increasing the proportion of potassium increases the tendency for the crystals to have rough basal planes, and hence an increase in the height/length ratio.

The inclusion of a divalent cation source in the zeolite synthesis mixture encourages the formation of flat basal planes and small crystals of low l/d ratio, and reduces the formation of crystalline contaminants such as zeolite W and erionite.

The amount of divalent cation which should be present in the synthesis mixture depends on the particular cation. However, in general up to 250 ppm based on the weight of the synthesis gel is used. Barium may be used in an amount up to 250 ppm, but an advantageous effect is seen when much smaller amounts, such as 100 ppm, are used. Magnesium, on the other hand, need only be present in an amount of about 10 ppm to obtain hockeypuck shaped crystals. Although a source of silica, for example, may contain e.g. magnesium as an impurity it has been found that such silica does not produce the same advantageous effect as when the magnesium or other cation is added to the synthesis mixture from a separate source.

The temperature at which the gel is heated to produce the zeolite also affects the morphology of the crystals produced. If the temperature is reduced then there is more nucleation, producing smaller crystals which have small channel lengths and hence are desirable. However, there is also a tendency for the crystals to have rough domed basal planes so that instead of the crystals being flat cylinders they are clam-like in shape. The crystallization temperature should therefore be chosen with a view to obtaining crystals of as small a size as is reasonable whilst maintaining the desired crystal shape. Typical temperatures used to obtain crystals of the desired shape are 150 to 200°C.

Accordingly, the proportions of the synthesis ingredients substances and the crystallization temperature should be adjusted to obtain the necessary dimensions e.g. length, diameter and shape of crystals, and the proportions and amounts specified above and in the examples are given for guidance.

The zeolite of type KL may be prepared by simple adaptation of techniques known in the art for producing zeolites. For example a source of silica and a source of divalent cations may be mixed with an aqueous solution of an alumina source and a K₂O source, to form a gel and this gel heated to form the zeolite crystals. Typically the gel is heated at 150 to 200°C for a period long enough to form the crystals. This is generally from 60 to 172 hours, typically between 60 and 160, preferably 60 to 150 hours. In general the lower the temperature the longer the time required to reach the same degree of crystallisation with the same synthesis mixture.

The source of silica may be e.g. solid silica or an aqueous solution or colloid of silica such as that sold under the trade name "Ludox" available from E.I. Dupont de Nemours & Co. Colloidal Sols are preferred since they result in less contaminating phases. However other forms such as silicates may be used. The source of divalent cations may be provided in the form of a powder or a solution, e.g. an aqueous solution of an alkaline earth metal hydroxide.

The source of alumina may be an alumina introduced into the synthesis mixture as e.g. Al₂O₃. 3H₂O previously dissolved in alkali. It is also possible to introduce a source of alumina into the synthesis mixture in the form of aluminium metal dissolved in alkali.

The source of K₂O is preferably introduced into the synthesis mixture as potassium hydroxide.

During the production of zeolite KL, stirring the synthesis mixture during heating increases nucleation and therefore speeds up the formation of crystals and encourages the formation of smaller crystals. However, this has the disadvantage that it also encourages the formation of the undesirable contaminant, zeolite W. Inclusion of a divalent metal cation according to the present invention allows the synthesis mixture to be stirred during crystallization but suppresses the formation of zeolite W.

The aluminosilicate forms of the invention may be hydrated, typically with from 0 to 9 moles of water per mole of Al₂O₃. When used as a catalyst base, the zeolite prepared by the invention is preferably first calcined to remove water. In normal preparation from aqueous gels a hydrated form is first prepared and this may be dehydrated by heating.

The product of the process is predominantly a potassium form of the aluminosilicate. By ion exchange of the product in the manner well-known to zeolite chemistry, other cations such an Na or H can be introduced in place of the potassium.

The zeolite may be treated in the same way as conventional zeolites L to improve its mechanical strength e.g. by forming an extrudate.

A catalyst based on the zeolite may be formed by impregnating or "loading" the zeolite with a metal which promotes the desired reaction e.g. aromatization. The metal is preferably platinum or a mixture of platinum and at least one other metal such as tin, germanium, rhenium or iridium. The total amount of metal loaded on the zeolite is typically 0.4 to 0.8 weight % based on the weight of the zeolite, preferably about 0.6 weight %. The loading may be carried out by processes known in the art.

### DESCRIPTION OF THE FIGURES

Reference is made in the examples to five figures:
Figure 1A and 1B show a scanning electron micrograph (SEM) of "hockeypuck" zeolite crystals.
Figure 2A and 2B show a SEM of comparative zeolite crystals which are not of the desired shape.
Figure 3A and 3B show SEMs of "hockeypuck" zeolite crystals.
Figure 4 shows SEMs of "hockeypuck" zeolite crystals made in a large volume and a small volume synthesis.
Figure 5A and 5B show the benzene yield and selectivity for three catalysts.

### EXAMPLES

The following examples illustrate the invention:

### EXAMPLE 1:

Preparation synthesis mixture (weight of reactants are given in grams).

| POTASSIUM ALUMINATE SOLUTION: | |
|---|---|
| KOH pellets (86.8% purity) | 34.30 |
| Al (OH)₃, (98.6% purity) | 7.91 |
| H₂O | 50.10 |
| Rinse water | 25.00 |

| Silicate Solution: | |
|---|---|
| Collodial Silica (Ludox HS-40*) | 150.26 |
| Ba(OH)₂ 8 H₂O Crystals | 0.0999 |
| H₂O | 50.01 |
| Rinse water | 64.47 |

| | |
|---|---|
| * Ludox HS-40 is a collodial Silica of DUPONT. | |

The alumina was dissolved in the KOH solution by boiling. The solution was cooled to room temperature and corrected for weight loss.

The Ba-source was dissolved in a portion of the water and was added to the Ludox together with another portion of the water which was used to rinse the beaker containing the Ba-source. The resulting solution was stirred for 5 minutes. Next the aluminate solution including the rinse water was added and the whole was mixed for another 3 minutes.

The composition of the synthesis mixture was:
2.65 K₂O/0.0032 BaO/0.5 Al₂O₃/10 SiO₂/159 H₂O

This corresponds to 115 ppm Ba⁺⁺ based on the weight of the gel. 323.10 g of the synthesis mixture was transferred to a 300 ml stainless steel autoclave. The autoclave was placed in an oven and heated up to 170°C and was kept at this temperature for 96 hours.

The product was separated from the mother liquor by centrifuging. It was washed to pH 9.7 and dried overnight at 150°C. The weight of the recovered product was 25.1 gram.

The product was analyzed using x-ray diffraction (XRD), Scanning Electron Micrographs (SEM), and Toluene Adsorption Measurement (TGA) with the following results:
- XRD :: pure KL, crystallinity vs standard: 92%
- SEM :: flat crystals with microscopically flat basal planes,
Length : ⁻0.20 microns;
diameter : ⁻0.60 microns;
l/d ratio : ⁻0.3;
height/length (h/l) ratio : 1.
- TGA :: wt % toluene adsorption at p/po = 0.25, T = 30°C:10.6.

### EXAMPLE 2: (Comparative):

Synthesis without added divalent cations.

An identical synthesis mixture was prepared as in example 1, but in this case no Ba was added to the synthesis mixture used. The synthesis mixture was crystallized for 96 hours at 170°C. The product was analyzed by XRD and SEM with the following results:
- XRD :: the product was partially crystalline, e.g. contained amorphous gel particles and was contaminated with an Erionite-like crystalline phase. Crystallinity vs standard : 45%.
- SEM :: micrographs showed the presence of amorphous gel particles and other contaminants. The KL crystal had a low l/d ratio but the crystals were relatively large and the basal planes showed terraces and step growth. The crystallite dimensions were:
Length : ⁻1.5 microns;
diameter : ⁻4.5 microns;
l/d ratio : ⁻0.3.

From these results can be seen that this experiment did not produce the KL-product of the invention.

### EXAMPLE 3:

Variation in the source of divalent cation.

An identical synthesis mixture was prepared as in example 1 but in this case the synthesis mixture was seeded with 9 ppm Mg²⁺ (based on the weight of the synthesis mixture). The Mg²⁺ source was Mg(NO₃)₂. 6 H₂O. The synthesis mixture was crystallized at 170°C for 96 hours. The resulting product was analyzed by XRD, SEM and TGA with the following results.
- XRD :: pure KL, crystallinity vs standard: 97%
- SEM :: flat KL crystals with microscopically flat basal planes,
Length : 0.1 ⁻ 0.4 microns;
diameter : 0.4 - 0.8 microns;
l/d ratio : ⁻0.4;
height/length (h/l) ratio : 1.
- TGA :: wt % toluene adsorption: 10.5.

Examples 4 and 5: variation in the K₂O content of the synthesis mixture.

### EXAMPLE 4: (Comparative):

This shows the effect of increased K₂O level in the synthesis mixture. A synthesis mixture was prepared in the same way as in Example 1 but with a molar composition of:
3.00 K₂O/0.0064 BaO/0.50 Al₂O₃/10 SiO₂/160 H₂O

This mixture was crystallized for 72 hours at 170°C. The resulting product was analyzed by XRD, SEM and TGA with the following results:
- XRD :: pure KL, crystallinity vs standard: 76%
- SEM :: flat KL crystals with terraces on the basal planes,
Length : ⁻0.15 microns;
diameter : ⁻0.15 - 0.3 microns;
l/d ratio : ⁻0.4;
height/length (h/l) ratio : >1.
- TGA :: wt % toluene adsorption: 11.0.

This did not give the crystals of the invention since the basal planes were not sufficiently flat.

### EXAMPLE 5:

A synthesis mixture was prepared in the same way as in Example 1 but with a molar composition of:
2.40 K₂O/0.0064 BaO/0.50 Al₂O₃/10 SiO₂/159 H₂O
i.e. reducing the alkalinity to the region of its lowest limit.

The mixture was crystallized for 96 hours and for 144 hours at 170°C. The product obtained after 96 hours had a low XRD-crystallinity (53% vs standard) and contained amorphous gel particles. The product after 144 hours crystallization still had a low XRD crystallinity (67% vs standard) and was slightly contaminated with an Erionite-like crystalline phase. The 144 hours - product consisted of flat KL crystals with microscopically flat basal planes. The particle size distribution was significantly increased. Crystallite dimensions:
- Length :: 0.2 - 0.8 microns
- Diameter :: 0.3 - 1.0 microns
- l/d ratio :: 0.2 - 0.6.

Further examples were carried out in which various parameters were varied in the compositions and their preparations. Table 1 gives details of the synthesis of the various zeolites and Table 2 gives details of the characteristics of the resulting products. Examples 1, 3, 5, 8, 9, 11 to 13 and 15 illustrate the invention.

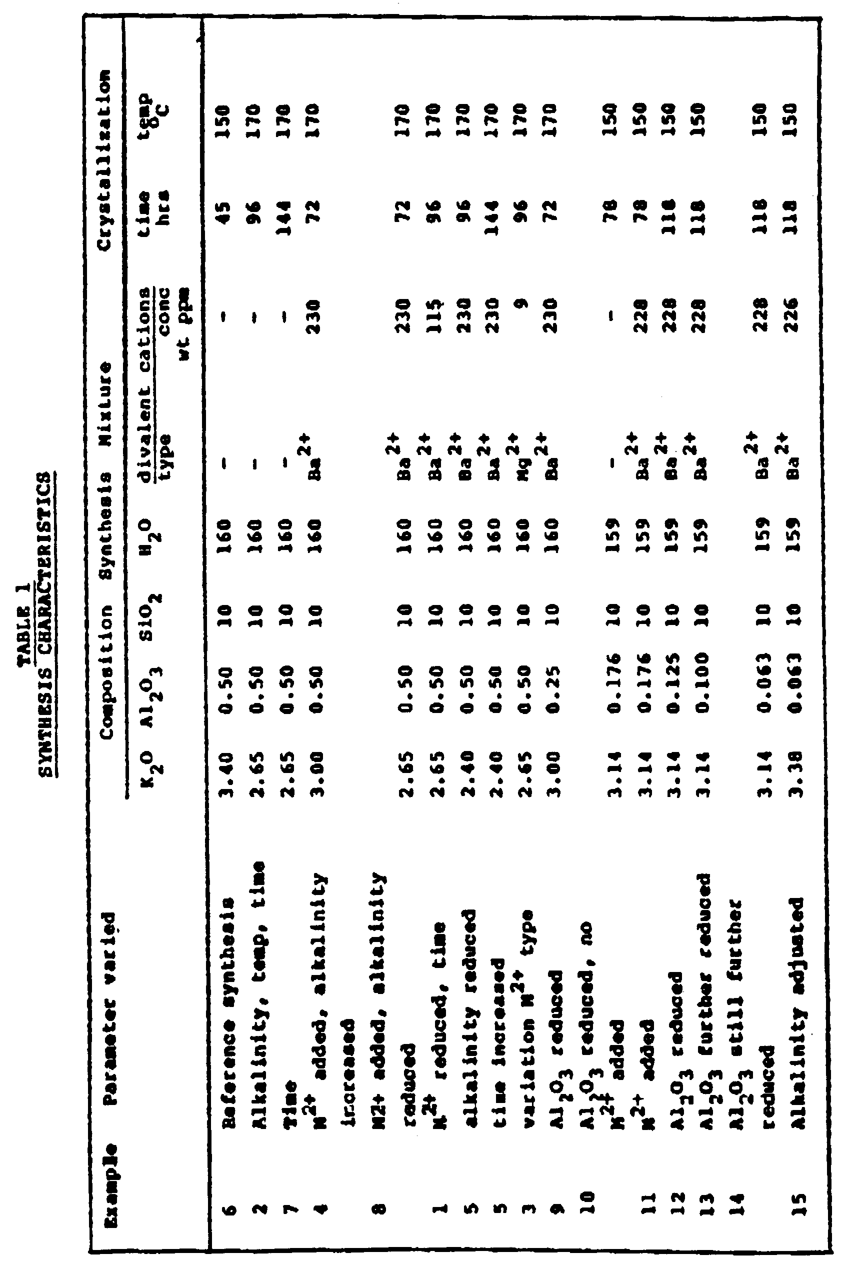

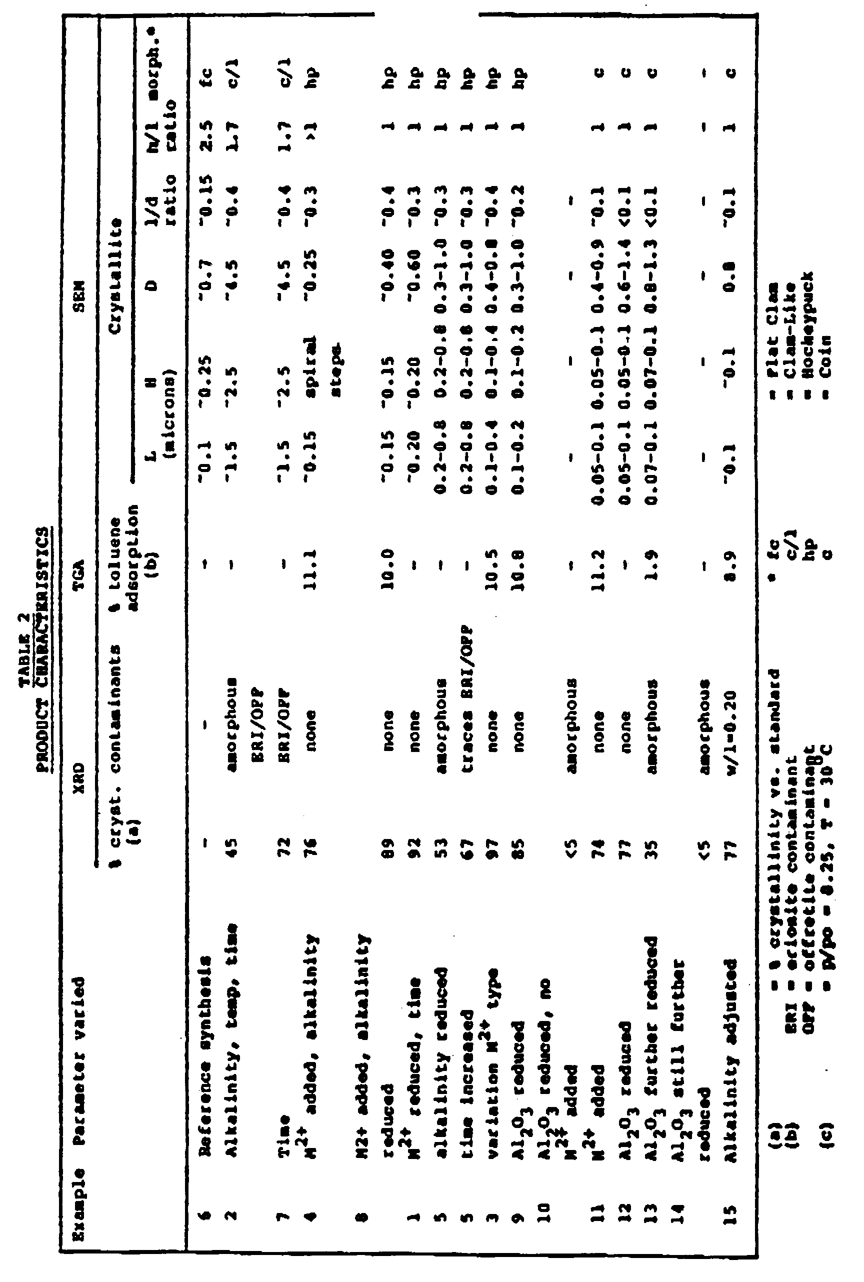

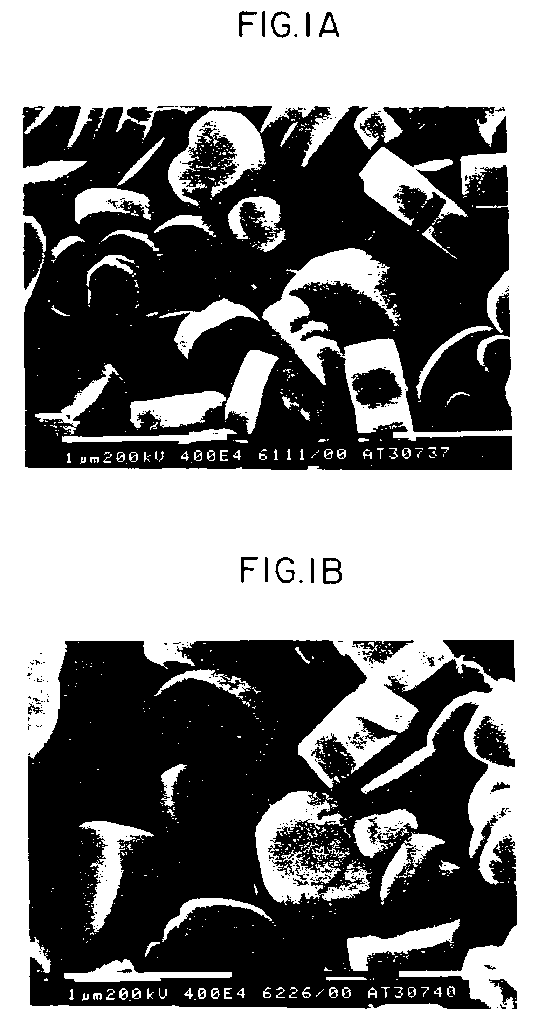

Figure 1A shows scanning electron micrographs of the crystals of zeolite prepared in Example 1 (using Ba²⁺ as the cation). Figure 1B shows scanning electron micrographs of crystals of zeolite prepared in Example 3 (using Mg²⁺ as the cation). The magnification of Figures 1A and 1B is 40000 times.

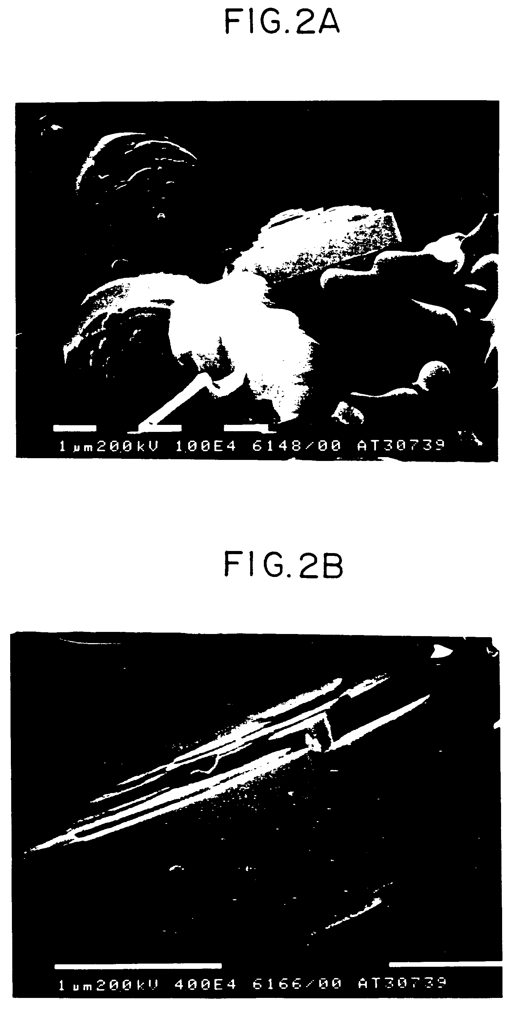

Figures 2A and 2B show scanning electron micrographs of the crystals of zeolite prepared in Example 2 in which no divalent cation was used. The magnification of Figure 2A is 10000 times. The magnification of Figure 2B is 40000 times. A comparison of Figure 2A and 2B with Figure 1A and 1B shows that the crystals of Example 2 are much larger and do not have flat basal planes.

The wavy lines in the right half of Figure 2A and 2B show the contamination by amorphous particles of unreacted gel.

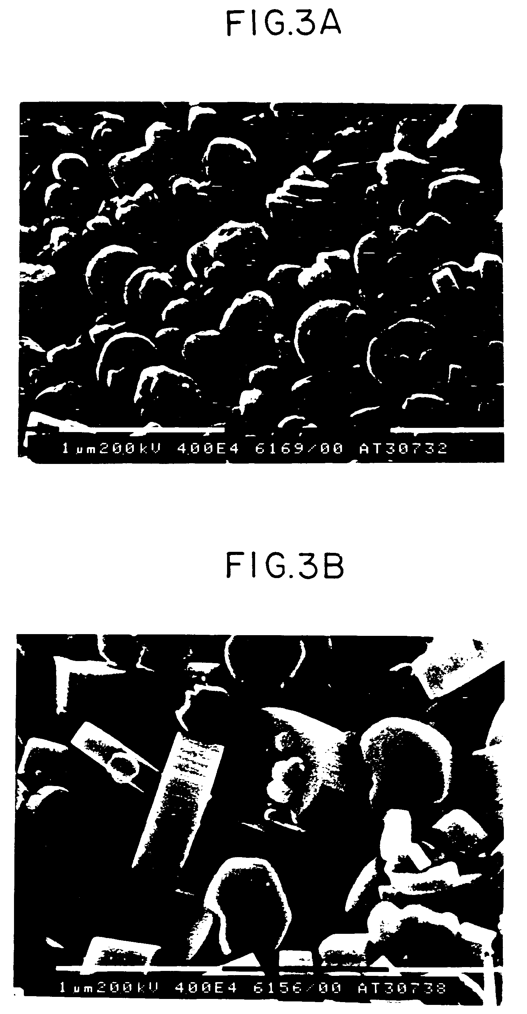

Figure 3A shows scanning electron micrographs of the crystals of zeolite prepared in Example 4. Figure 3B shows scanning electron micrographs of the crystal of zeolite prepared in Example 5. It can be seen that the crystals of Example 4, in which the K20 content is increased, are not flat and have terraces on the basal planes. The magnification of Figures 3A and 3B is 40000 times.

### Example 16:

This example illustrates the use of Mg²⁺ as the divalent cation, and demonstrates that the slower heating up of a gel which would be a feature of large scale production can be used successfully to produce crystals of the desired shape and dimensions.

25 litres synthesis. Preparation synthesis mixture (weight of reactants given in grams):

| | | |
|---|---|---|
| (A) | KOH (87.7% purity) | 1878.06 |
| | Al(OH)₃ (99.3% purity) | 433.07 |
| | H₂O | 3154±5 |
| | Rinse Water | 420.0 |
| (B) | Ludox HS-40 | 8250±5 |
| | Mg(NO₃)₂6H₂O | 2.0484 |
| | H₂O | 1799.67 |
| | H₂O | 4640±5 |
| | Rinse Water | 420.0 |

### SOLUTION A:

The ingredients were dissolved with boiling water under reflux in a 6 litre pyrex bottle and the solution cooled to room temperature.

### SOLUTION B:

The Mg²⁺ source was dissolved in 1799.67 grams of the water. In a separate 25 litre polypropylene flask the Ludox was diluted with 4640 grams of water and this solution was poured into the autoclave. The polypropylene flask was rinsed with 420 grams of water and the rinse water added to the autoclave. The Mg²⁺ solution was then poured into the diluted Ludox solution in the autoclave and the whole was mixed for 5 minutes.

Solution A was then added and mixing was continued for a further five minutes. A thick, smooth gel was obtained.

The gel composition was:
2.67 K₂O/0.50 Al₂O₃/10 SiO₂/160 H₂O + 9 ppm Mg²⁺

The gel was heated up over 10 hours to 170°C although it took approximately 13 hours for the centre of the autoclave to reach 170°C. The autoclave was maintained at 170°C for 93 hours.

Before heating the gel a small sample (123.77 grams) of the gel was removed and crystallized separately as a satellite batch in an oven.

After crystallization a sample was taken from the main batch, washed to pH10.2 and the product was dried for 6 hours at 126°C and 16 hours at 150°C. The weight of product recovered was 200 grams.

Samples from the main and satellite batches were analysed. X-ray diffraction showed the crystallization of the main batch to be 95% compared with the standard, and the crystallization of the satellite sample to be 96% compared to the standard.

Both products were very slightly contaminated with erionite.

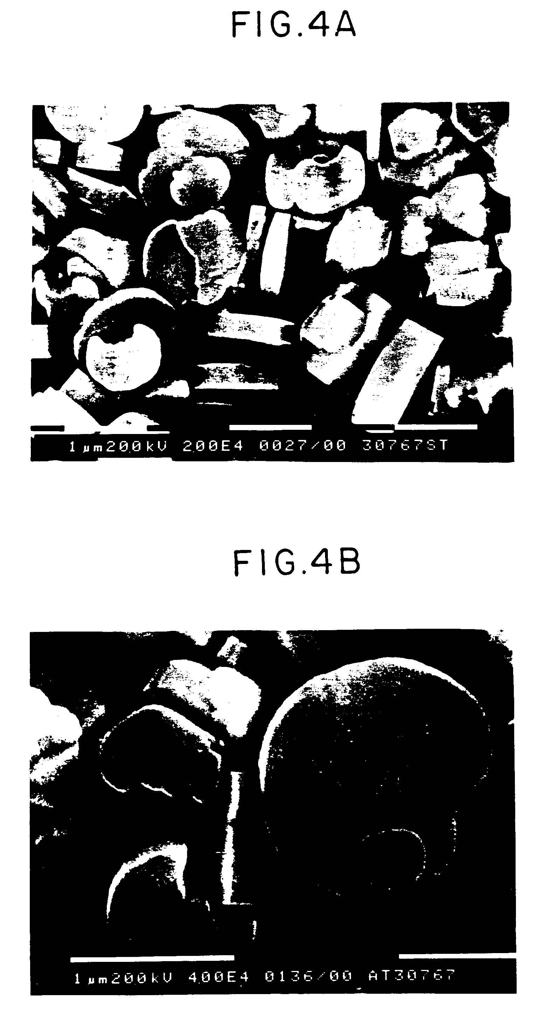

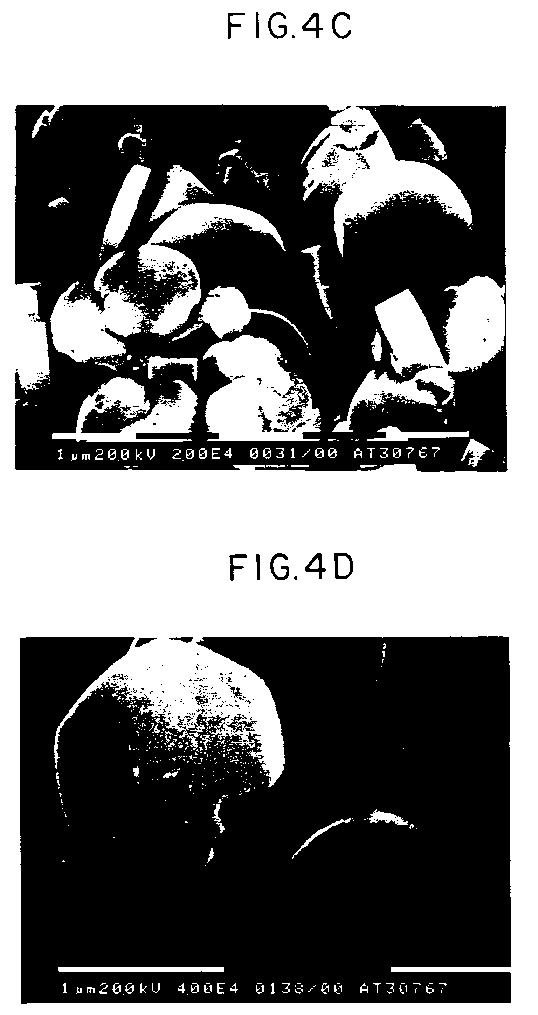

Figure 4(A), 4(B), 4(C) and 4(D) show the scanning electron micrographs of the crystals of zeolite prepared according to this example. Figures 4A and 4B are the satellite batch. Figures 4C and 4D are the batch crystallized in the 25 litre autoclave. Figure 4A and 4C are at magnification 20000 times, and Figure 4B and 4D are at magnification 40000. It can be seen that both batches give hockeypuck shaped crystals with flat basal planes.

### EXAMPLE 17:

The effectiveness of the zeolite KL of the present invention as a base for an aromatization catalyst was compared with that of a reference zeolite KL which was a zeolite KL prepared according to EP-A-96749. Using similar techniques to the previous examples the following zeolites were prepared, and loaded according to known techniques with nominally 0.6 wt % Pt.

| ZEOLITE | ZEOLITE TYPE | LENGTH (microns) | DIAMETER (microns) |
|---|---|---|---|
| Reference | Standard KL Type | 0.8-1.3 | 1.1-1.3 |
| Example 8 | Ba added. Hockeypuck crystals with flat basal planes | 0.1-0.2 | 0.4-0.6 |
| Example 16 | Mg added Hockeypuck crystals with flat basal planes | 0.2-0.6 | 0.6-1.5 |

A feedstock of 54% 3-methylpentane, 36% n-hexane, and 10% methylcyclopentane was subjected in the presence of each of the above zeolite catalysts to a temperature of 510°C and 135 psig (930 kPa) total pressure for 170 hours. The H₂/Feed ratio was 4.

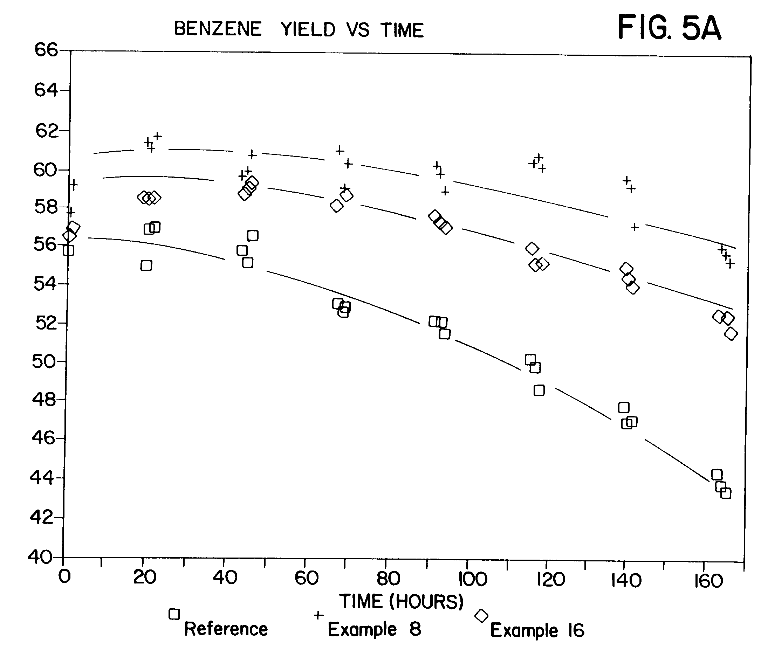

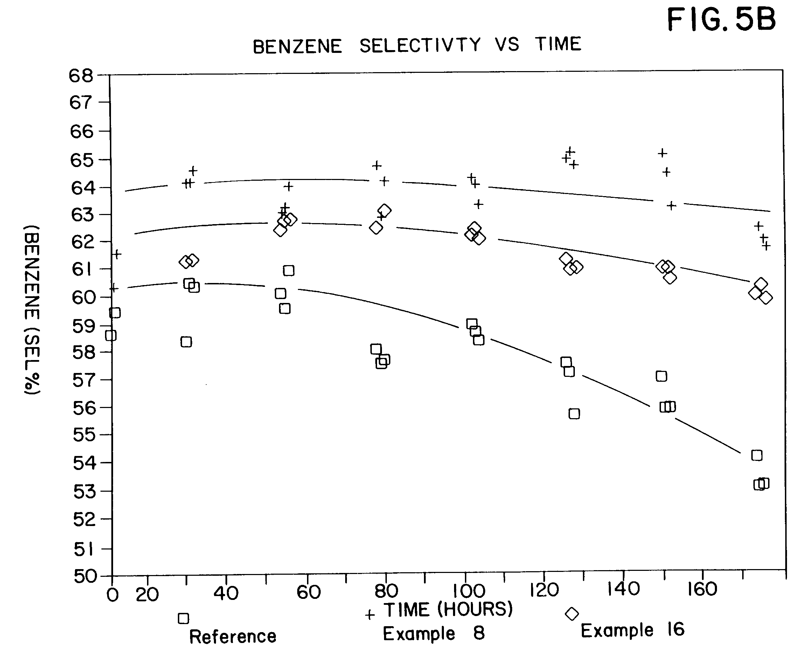

Figure 5(A) shows the benzene yield versus time for the three catalysts and Figure 5(B) shows benzene selectivity vs. time for the three catalysts. It can be seen that the very flat cylindrical (hockeypuck) crystals give a better yield and selectivity. Regression analysis performed on the data obtained gave the following results:-

| TIME AVERAGE VALUE (92 HOURS) | | | | |
|---|---|---|---|---|
| CATALYST | YIELD | SELECTIVITY | CONVERSION | CYCLE LENGTH (hr) at 38% TAY |
| REFERENCE | 52.2 | 58.9 | 88.0 | 2056 |
| EXAMPLE 8 | 60.2 | 64.3 | 93.0 | 7982 |

TAY is the Time Average Yield. This is a measure of the time over which the catalyst produces a benzene yield of at least 38%. The higher the TAY the longer the catalyst functions before it needs to be regenerated.

It can be seen that the improved stability of the "hockeypuck" catalyst gives a greater selectivity and yield and also allows for a greatly increased cycle length compared with the reference catalyst of KL type.

## Claims

1. A Zeolite L in which the crystals are cylindrical and have an average length of 0.6 microns or less and an average length: diameter ratio of less than 0.5 and an average height: length ratio of 1 to 1.2.

2. A zeolite as claimed in claim 1 in which the average height: length is approximately 1.

3. A process for producing a zeolite L in which the crystals are cylindrical and have an average length of 0.6 microns or less and an average length: diameter ratio of less than 0.5 and have substantially flat basal planes, which process comprises mixing an aqueous solution of silica and divalent cation with an aqueous solution of alumina and KOH, and heating mixture to crystallise the desired zeolite.

4. A process as claimed in claim 3 in which the mixture comprises q moles of water, a divalent cation, a source of m moles of K₂O, a source of n moles of SiO₂ and a source of p moles of Al₂O₃ wherein m:n is 0.2 to 0.35 and n:p is 15 to 160 and q:m is 45 to 70.

5. A process as claimed in claim 4 where m:n is 0.24 to 0.30 and n:p is 20 to 40 and q:m is 50 to 65.

6. A process as claimed in claim 4 and 5, in which the divalent cation is nickel, magnesium, calcium, barium, cobalt, manganese, zinc, copper or tin.

7. A process as claimed in claim 6, in which the divalent cation comprises up to 250 ppm of the weight of the mixture.

8. A process according to any of claims 3 to 7 in which the mixture is heated to 150 to 200°C.

9. A process according to claim 8 in which the mixture is heated for 60 to 160 hours.

## Patentansprüche

1. Zeolith L, bei dem die Kristalle zylindrisch sind und eine durchschnittliche Länge von 0,6 µm oder weniger und ein mittleres Länge:Durchmesser-Verhältnis von weniger als 0,5 und ein mittleres Höhe:Länge-Verhältnis von 1 bis 1,2 aufweisen.

2. Zeolith nach Anspruch 1, bei dem das mittlere Höhe:Länge-Verhältnis etwa 1 ist.

3. Verfahren zur Herstellung eines Zeolith L, bei dem die Kristalle zylindrisch sind und eine mittlere Länge von 0,6 µm oder weniger und ein mittleres Länge:Durchmesser-Verhältnis von weniger als 0,5 und im wesentlichen flache Basisflächen aufweisen, bei dem eine wäßrige Lösung von Siliciumoxid und einem zweiwertigen Kation mit einer wäßrigen Lösung von Aluminiumoxid und KOH gemischt werden und die Mischung erwärmt wird, um den gewünschten Zeolith zu kristallisieren.

4. Verfahren nach Anspruch 3, bei dem die Mischung q Mole Wasser, ein zweiwertiges Kation, eine Quelle für m Mole K₂O, eine Quelle für n Mole SiO₂ und eine Quelle für p Mole Al₂O₃ umfaßt, wobei m:n 0,2 bis 0,35, n:p 15 bis 160 und q:m 45 bis 70 beträgt.

5. Verfahren nach Anspruch 4, bei dem m:n 0,24 bis 0,30, n:p 20 bis 40 und q:m 50 bis 65 ist.

6. Verfahren nach Anspruch 4 oder 5, bei dem das zweiwertige Kation Nickel, Magnesium, Calcium, Barium, Kobalt, Mangan, Zink, Kupfer oder Zinn ist.

7. Verfahren nach Anspruch 6, bei dem das zweiwertige Kation bis zu 250 ppm des Gewichts der Mischung ausmacht.

8. Verfahren nach einem der Ansprüche 3 bis 7, bei dem die Mischung auf 150 bis 200 °C erwärmt wird.

9. Verfahren nach Anspruch 8, bei dem die Mischung 60 bis 160 Stunden erwärmt wird.

## Revendications

1. Zéolite L dans laquelle les cristaux sont cylindriques et ont une longueur moyenne égale ou inférieure à 0,6 micromètre, un rapport longueur moyenne:diamètre inférieur à 0,5 et un rapport hauteur moyenne:longueur de 1 à 1,2.

2. Zéolite suivant la revendication 1, dans laquelle le rapport hauteur moyenne:longueur est approximativement égal à 1.

3. Procédé pour la production d'une zéolite L dans laquelle les cristaux sont cylindriques et ont une longueur moyenne égale ou inférieure à 0,6 micromètre, un rapport longueur moyenne:diamètre inférieur à 0,5 et des plans de base essentiellement plats, procédé qui comprend le mélange d'une solution aqueuse de silice et d'un cation divalent à une solution aqueuse d'alumine et de KOH, et le chauffage du mélange pour provoquer la cristallisation de la zéolite désirée.

4. Procédé suivant la revendication 3, dans lequel le mélange comprend q moles d'eau, un cation divalent, une source de m moles de K₂O, une source de n moles de SiO₂ et une source de p moles de Al₂O₃, le rapport m:n ayant une valeur de 0,2 à 0,35, le rapport n:p ayant une valeur de 15 à 160 et le rapport q:m ayant une valeur de 45 à 70.

5. Procédé suivant la revendication 4, dans lequel le rapport m:n a une valeur de 0,24 à 0,30, le rapport n:p a une valeur de 20 à 40 et le rapport q:m a une valeur de 50 à 65.

6. Procédé suivant les revendications 4 et 5, dans lequel le cation divalent est le nickel, le magnésium, le calcium, le baryum, le cobalt, le manganèse, le zinc, le cuivre ou l'étain.

7. Procédé suivant la revendication 6, dans lequel le cation divalent représente jusqu'à 250 ppm du poids du mélange.

8. Procédé suivant l'une quelconque des revendications 3 à 7, dans lequel le mélange est chauffé à une température de 150 à 200°C.

9. Procédé suivant la revendication 8, dans lequel le mélange est chauffé pendant un temps de 60 à 160 heures.
